# EUROPEAN PATENT SPECIFICATION

(11) **EP 2 054 011 B1**
(45) Date of publication and mention of the grant of the patent: **19.09.2018**
(21) Application number: 07766841.6
(22) Date of filing: 01.07.2007
(51) Int. Cl.: A61K 8/22, A61K 8/38, A61Q 11/00

(54) **METHOD AND APPARATUS FOR ELECTROCHEMICALLY WHITENING TEETH**
VERFAHREN UND VORRICHTUNG ZUM ELEKTROCHEMISCHEN BLEICHEN VON ZÄHNEN
BLANCHIMENT ÉLECTROCHIMIQUE DES DENTS

(30) Priority: 29.06.2006 US 476787
(43) Date of publication of application: 06.05.2009
(73) Proprietor: Colgate-Palmolive Company, New York, NY 10022 (US)
(72) Inventor: KHAWALED, Kamal, 20200 Shfaram (IL); JUBEH, Maher, 97200 Jerusalem (IL)
(74) Representative: Thum, Bernhard
(86) International application number: PCT/IL2007/000811
(87) International publication number: WO 2008/001388

(56) References cited:
- EP-A- 1 457 200
- EP-A- 1 525 857
- WO-A-2005/062710
- WO-A-2007/047568
- AU-A1- 2006 202 776
- FR-A- 2 873 297
- US-A- 3 215 139

## Description

### FIELD AND BACKGROUND OF THE INVENTION

The present invention relates to electrochemically whitening teeth, and more particularly, to a device for electrochemically whitening teeth. The present invention is based on applying an oxidizing agent to teeth, combined with transmitting a safe, low level electrical current directly to the teeth, for a short period of time, for effecting the teeth whitening (via electrochemical activation and redox reactions). Implementation of the present invention provides excellent control of safely applying, delivering, or dispensing, of a teeth whitening oxidizing agent to teeth, and involves minimal exposure of non-tooth intra-oral cavity components, e.g., gum and tissue exterior surfaces, to the oxidizing agent. The present invention is simple and inexpensive to manufacture, and is simple, safe, and inexpensive, to implement either by a dental health provider alongside a subject to be treated in a dental health office, or by a subject itself outside of a dental health office, and is commercially applicable.

### Whitening teeth (teeth whitening)

In the context of the field and art of the present invention, 'whitening teeth' (or teeth whitening) generally refers to application of any number of chemical (e.g., oxidation, reduction) or/and physical (e.g., mechanical, thermal, irradiation, electrical) processes, procedures, techniques, or treatments, performed singly or in combination, which results in returning, or/and providing, white color to teeth (i.e., to a single tooth, or to two or more teeth).

There exists a plethora of prior art teachings of a wide variety of different chemical or/and physical processes, procedures, techniques, or treatments, performed singly or in combination, for whitening teeth, where each prior art teaching features different particular advantages and disadvantages. For example, use of hydrogen peroxide, or/and other relatively strong oxidizing agents, at appropriate concentrations and conditions of oral application or treatment, for whitening teeth, are most commonly taught about and practiced. During such 'chemical oxidation' type teeth whitening techniques, teeth are exposed to the oxidizing agent(s), whereby the oxidizing agent(s) is/are reduced, and in turn, oxidize, and possibly also chemically degrade, various substances (mostly composed of organic matter) which discolor teeth. However, applications of such techniques for whitening teeth are typically accompanied by inadvertent or unpreventable, and undesirable, exposure of non-tooth intra-oral cavity components, e.g., gum and tissue exterior surfaces, to the oxidizing agent(s), or/and to the means used for applying, delivering, or dispensing, the oxidizing agent(s) to the teeth. Additionally, applications of such techniques for whitening teeth usually require implementation by a dental health provider alongside a subject to be treated in a dental health office, and are usually not implementable by a subject itself outside of a dental health office. In spite of the vast amounts of prior art teachings of using oxidizing agents in techniques for whitening teeth, there remains on on-going need for improving existing techniques, and identifying new techniques, which improve control of applying, delivering, or dispensing, of teeth whitening oxidizing agents to teeth, and which involve minimal exposure of non-tooth intra-oral cavity components, e.g., gum and tissue exterior surfaces, to the oxidizing agents, as well as to the means used for applying, delivering, or dispensing, the oxidizing agents to the teeth.

There is thus a widely recognized need for, and it would be highly advantageous to have, a method and device for electrochemically whitening teeth, which are devoid of the above stated, and of various other, limitations and disadvantages. There is also need for such an invention whose implementation provides excellent control of safely applying, delivering, or dispensing, of a teeth whitening oxidizing agent to teeth, and involves minimal exposure of non-tooth intra-oral cavity components, e.g., gum and tissue exterior surfaces, to the oxidizing agent Moreover, there is need for such an invention which is simple and inexpensive to manufacture, which is simple, safe, and inexpensive, to implement either by a dental health provider alongside a subject to be treated in a dental health office, or by a subject itself outside of a dental health office, and which is commercially applicable.

WO 2005/062710 A2 describes a pre-treatment tray with a sealing lip. EP 1 525 857 A1 describes a tray for treating teeth.

### SUMMARY OF THE INVENTION

The present invention relates to electrochemically whitening teeth, and more particularly, to a device for electrochemically whitening teeth as defined in claim 1. The present invention is based on applying an oxidizing agent to teeth, combined with transmitting a safe, low level electrical current directly to the teeth, for a short period of time, for effecting the teeth whitening (via electrochemical activation and redox reactions). Implementation of the present invention provides excellent control of safely applying, delivering, or dispensing, of a teeth whitening oxidizing agent to teeth, and involves minimal exposure of non-tooth intra-oral cavity components, e.g., gum and tissue exterior surfaces, to the oxidizing agent. The present invention is simple and inexpensive to manufacture, and is simple, safe, and inexpensive, to implement either by a dental health provider alongside a subject to be treated in a dental health office, or by a subject itself outside of a dental health office, and is commercially applicable.

According to an example useful for understanding the present invention, there is provided a method for electrochemically whitening teeth, the method comprising: applying a metal salt solution to the teeth, applying an oxidizing agent to the teeth exposed to the metal salt solution, and transmitting electrical current to the teeth so as to activate and reduce the oxidizing agent for effecting the whitening of the teeth.

According to the present invention, there is provided a device for electrochemically whitening teeth, the device comprising: an applicator suitable for applying a substance to the teeth, the applicator having a first end and a second end, a first electrode attached to the first end of the applicator, a second electrode attached to the second end of the applicator, wherein the first electrode and the second electrode are configured to transmit electrical current through the applicator, and an oxidizing agent suitable for placement within the applicator, wherein the oxidizing agent is configured to undergo activation and reduction upon transmission of the electrical current through the oxidizing agent.

According to further characteristics in preferred examples described below, the metal salt solution is selected from the group consisting of silver nitrate solution, palladium hydroxide solution, palladium chloride solution, titanium chloride solution, and copper chloride solution. According to further characteristics in preferred examples described below, applying the metal salt solution precedes applying the oxidizing agent and transmitting electrical current.

According to further characteristics in preferred examples useful for understanding the invention described below, there is rinsing of the teeth with distilled water following applying the metal salt solution.

According to further characteristics in preferred examples useful for understanding the invention described below, applying the metal salt solution is done using a pre-treatment tray, the pre-treatment tray designed to prevent leakage of the metal salt solution into a mouth.

According to further characteristics in preferred examples useful for understanding the invention described below, applying the oxidizing agent includes applying a teeth whitening agent.

According to further characteristics in preferred embodiments of the invention described below, the teeth whitening agent includes hydrogen peroxide.

According to further characteristics in preferred embodiments of the invention described below, the oxidizing agent is selected from the group consisting of a whitening solution, a whitening gel, a solution of or including carbamide peroxide, a gel of or including carbamide peroxide, a solution of or including sodium perborate, and a gel of or including sodium perborate.

According to further characteristics in preferred examples useful for understanding the invention described below, transmitting electrical current is done at a voltage in a range of between about 0.001 volt and about 12 volts, and more preferably, in a range of between about 3 volts and about 9 volts.

According to further characteristics in preferred examples useful for understanding the invention described below, transmitting electrical current is done for the electrical current in a range of between about 0.001 milliamp (mA) and about 30 milliamps (mA).

According to further characteristics in preferred examples useful for understanding the invention described below, transmitting electrical current is done for the electrical current in a range of between about 5 milliamp (mA) and about 15 milliamps (mA).

According to further characteristics in preferred examples useful for understanding the invention described below, transmitting electrical current is done by a rechargeable battery, or alternatively, by a disposable battery embedded into a device for applying the oxidizing agent. According to the invention described below, the applicator is a dental tray.

According to further characteristics in preferred examples useful for understanding the invention described below, the applicator is a toothbrush.
According to further characteristics in preferred embodiments of the invention described below, the first end is a back curved wall and the first electrode is positively charged.

According to further characteristics in preferred embodiments of the invention described below, the first electrode is a flat metal strip.

According to further characteristics in preferred embodiments of the invention described below, the device further comprises spacers on the first electrode to separate the first electrode from the teeth.

According to further characteristics in preferred embodiments of the invention described below, the spacers are comprised of a biocompatible plastic material.

According to further characteristics in preferred embodiments of the invention described below, the second end is a front wall and the second electrode is negatively charged.

According to further characteristics in preferred embodiments of the invention described below, the second electrode further comprises a spring mechanism.

According to further characteristics in preferred embodiments of the invention described below, the front wall includes at least three negatively charged electrodes, and wherein each of the three electrodes is configured to contact at least part of a single tooth at a contact area on the part.

According to further characteristics in preferred embodiments of the invention described below, the contact area is minimized.

According to the invention described below, the dental tray further comprises a sealing lip having a zig-zag shape or contour.

According to further characteristics in preferred examples useful for understanding the invention described below, the device further comprises a power supply, being an external power source, or alternatively, a rechargeable battery, or alternatively, a disposable battery embedded within the applicator.

According to further characteristics in preferred examples useful for understanding the invention described below, transmission of the electrical current is done at a voltage in a range of between about 0.001 volt and about 12 volts.

According to further characteristics in preferred examples useful for understanding the invention described below, transmission of the electrical current is done at a voltage in a range of between about 3 volts and about 9 volts.

According to further characteristics in preferred examples useful for understanding the invention described below, transmission of the electrical current is done for said electrical current in a range of between about 0.001 milliamp (mA) and about 30 milliamps (mA). According to further characteristics in preferred examples useful for understanding the invention described below, transmission of the electrical current is done for said electrical current in a range of between about 5 milliamp (mA) and about 15 milliamps (mA).
Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

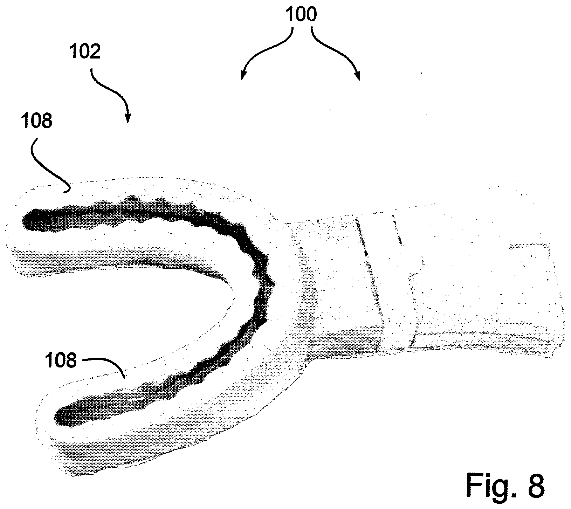

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. Only the figure showing a zig-zag contour is according to the invention (Figure 8).

In the drawings:
Fig. 1 is an isometric view of a device for electrochemically whitening teeth, in accordance with a preferred example useful for understanding the present invention;
Figs. 2a and 2b are cross-sectional illustrations of the electrochemical teeth whitening device of Fig. 1, in accordance with a preferred example useful for understanding the present invention; Figs. 3a and 3b are diagrammatic and cross-sectional views of a device for electrochemically whitening teeth, in accordance with another preferred example useful for understanding the present invention;
Figs. 4a and 4b are diagrammatic and cross-sectional views of an electrochemical teeth whitening device, in accordance with yet another preferred example useful for understanding the present invention;
Fig. 5 is a view of an electrochemical toothbrush for electrochemically whitening teeth, in accordance with another preferred example useful for understanding the present invention;
Figs. 6 and 7 are schematic diagrams illustrating perspective views of another exemplary preferred embodiment of an electrochemical device for electrochemically whitening teeth, showing the replaceable / rechargeable power supply in a connected or closed configuration (Fig. 6), and in a disconnected or open configuration (Fig. 7), in accordance with an example useful for understanding the present invention; and
Fig. 8 is a schematic diagram illustrating the electrochemical teeth whitening device shown in Figs. 6 and 7, particularly showing the sealing Hp with a 'zig-zag' shape or contour according to the invention.

### DESCRIPTION OF THE PREFERRED EMBODIMENTS

The present invention relates to electrochemically whitening teeth, and more particularly, to a device for electrochemically whitening teeth. The present disclosure is based on applying an oxidizing agent to teeth, combined with transmitting a safe low level electrical current directly to the teeth, for a short period of time, for effecting the teeth whitening (via electrochemical activation and redox reactions). Implementation of the present invention provides excellent control of safely applying, delivering, or dispensing, of a teeth whitening oxidizing agent to teeth, and involves minimal exposure of non-tooth intra-oral cavity components, e.g., gum and tissue exterior surfaces, to the oxidizing agent. The present invention is simple and inexpensive to manufacture, and is simple, safe, and inexpensive, to implement either by a dental health provider alongside a subject to be treated in a dental health office, or by a subject itself outside of a dental health office, and is commercially applicable.

A example useful for understanding the present invention is the provision of a method for electrochemically whitening teeth, including the following main procedures, and, components and functionalities thereof: applying a metal salt solution to the teeth, applying an oxidizing agent to the teeth, and transmitting electrical current to the teeth so as to activate and reduce the oxidizing agent for effecting the whitening of the teeth.

A main aspect of the present invention is the provision of a device for electrochemically whitening teeth with the features of claim 1, including the following main components and functionalities thereof: an applicator suitable for applying a substance to the teeth, the applicator having a first end and a second end, a first electrode attached to the first end of the applicator, a second electrode attached to the second end of the applicator, wherein the first electrode and the second electrode are configured to transmit electrical current through the applicator, and an oxidizing agent suitable for placement within the applicator, wherein the oxidizing agent is configured to undergo activation and reduction upon transmission of the electrical current through the oxidizing agent, thereby effecting the whitening of the teeth.

The present invention includes several aspects, and special technical features, of novelty and inventiveness over prior art teachings of whitening teeth.

A main aspect of the present disclosure is that structure and function, and subsequently, operation, of the electrochemical teeth whitening device, and implementation of the electrochemical teeth whitening method, are self-contained and localized with respect to the tooth or teeth being electrochemically whitened. Namely, the electrochemical circuit formed and activated during operation of the electrochemical teeth whitening device, for whitening teeth, does not require, or involve, any non-intra-oral cavity body part, for electrochemically applying, delivering, or dispensing, of teeth whitening oxidizing agents to the treated tooth or teeth.

Implementation of the present invention provides excellent control of safely applying, delivering, or dispensing, of a teeth whitening oxidizing agent to teeth, and involves minimal exposure of non-tooth intra-oral cavity components, e.g., gum and tissue exterior surfaces, to the oxidizing agent.

In addition to the preceding aspects of novelty and inventiveness, the present invention is simple and inexpensive to manufacture, and is simple, safe, and inexpensive, to implement either by a dental health provider in a dental health office, or by oneself outside of a dental health office.

It is to be understood that the present invention is not limited in its application to the details of the order or sequence, and number, of procedures, steps, and sub-steps, of operation or implementation of the method, or to the details of type, composition, construction, arrangement, order, and number, of the assemblies, sub-assemblies, mechanisms, structures, components, elements, and configurations, and, peripheral equipment, utilities, accessories, chemical reagents, and materials, of the device, set forth in the following illustrative description, accompanying drawings, and examples, unless otherwise specifically stated herein. Accordingly, the present invention is capable of other embodiments and of being practiced or carried out in various ways. Although procedures, steps, sub-steps, and system units, system sub-units, devices, assemblies, sub-assemblies, mechanisms, structures, components, elements, and configurations, and, peripheral equipment, utilities, accessories, chemical reagents, and materials, which are equivalent or similar to those illustratively described herein can be used for practicing or testing the present invention, suitable procedures, steps, sub-steps, and system units, system sub-units, devices, assemblies, sub-assemblies, mechanisms, structures, components, elements, and configurations, and, peripheral equipment, utilities, accessories, chemical reagents, and materials, are illustratively described and exemplified herein.

It is also to be understood that all technical and scientific words, terms, or/and phrases, used herein throughout the present disclosure have either the identical or similar meaning as commonly understood by one of ordinary skill in the art to which this invention belongs, unless otherwise specifically defined or stated herein. Phraseology, terminology, and, notation, employed herein throughout the present disclosure are for the purpose of description and should not be regarded as limiting. Additionally, as used herein, the term 'about' refers to ± 10 % of the associated value. Moreover, all technical and scientific words, terms, or/and phrases, introduced, defined, described, or/and exemplified, in the above Background section, are equally or similarly applicable in the illustrative description of the preferred embodiments, examples, and appended claims, of the present invention.

Procedures, steps, sub-steps, and, equipment and materials, assemblies, sub-assemblies, mechanisms, structures, components, elements, and configurations, and, peripheral equipment, utilities, accessories, chemical reagents, and materials, as well as operation and implementation, of exemplary preferred embodiments, alternative preferred embodiments, specific configurations, and, additional and optional aspects, characteristics, or features, thereof, of electrochemically whitening teeth, according to the present invention, are better understood with reference to the following illustrative description and accompanying drawings. Throughout the following illustrative description and accompanying drawings, same reference numbers, refer to same assemblies, sub-assemblies, mechanisms, structures, components, elements, and configurations, and, peripheral equipment, utilities, chemical reagents, accessories, and materials.

In the following illustrative description of the device of the present invention, included are main or principal procedures, steps, and sub-steps, and, main or principal assemblies, sub-assemblies, mechanisms, structures, components, elements, and configurations, and, peripheral equipment, utilities, accessories, chemical reagents, and materials, needed for sufficiently understanding proper 'enabling' utilization and implementation of the disclosed invention. Accordingly, description of various possible preliminary, intermediate, minor, or/and optional, procedures, steps, or/and sub-steps, or/and, system units, system sub-units, devices, assemblies, sub-assemblies, mechanisms, structures, components, elements, and configurations, and, peripheral equipment, utilities, accessories, chemical reagents, and materials, of secondary importance with respect to enabling implementation of the invention, which are readily known by one of ordinary skill in the art, or/and which are available in the prior art and technical literature relating to the field of the present invention, are at most only briefly indicated herein.

The present invention relates to a device for electrochemically whitening teeth.

Electrochemistry theory, principles, and practices thereof, and, related and associated applications and subjects thereof, are well known and taught about in the prior art, and currently practiced in a wide variety of numerous different fields and areas of technology. For the purpose of establishing the scope, meaning, and field(s) or area(s) of application, of the present invention, herein following are provided selected definitions and exemplary usages of terminology which are relevant to, and used for, disclosing the present invention.

### Electrochemistry - oxidation, reduction, and ionization, reactions

The term 'electrochemistry' is generally defined as the science that deals with the use of electrical energy to bring about a chemical reaction or with the generation of electrical energy by means of chemical action. Moreover, if a chemical reaction is caused by an external voltage, or if a voltage is caused by a chemical reaction, then, the chemical reaction is an electrochemical reaction.

In general, electrochemistry deals with oxidation, reduction, and ionization, types of chemical reactions. In general, an oxidation reaction is a chemical reaction which is based on the loss of an electron by a chemical species (atom, molecule, radical, or ion). In general, a reduction reaction is a chemical reaction which is based on the gain of an electron by a chemical species (atom, molecule, radical, or ion). A paired or complementary set of an oxidation reaction and a reduction reaction is commonly referred to as a 'redox' reaction. In general, ionization refers to the process of converting a chemical species (atom, molecule, radical) into an ion, and also refers to the state of a chemical species (atom, molecule, radical) being ionized, where an ion is an atom, a group of atoms, or a molecule, having a net (positive or negative) electrical charge. In general, an 'ionizable substance' refers to a substance which either is, or includes, a chemical species (atom, molecule, radical), that can be ionized, such that there is a change in the net electrical charge of the chemical species which is ionized.

In general, an Oxidizing agent', also known as an oxidizer or as an oxidant, refers to a chemical compound that readily gives up (atomic or/and molecular) oxygen. An oxidizing agent also refers to a substance that gains (receives) electrons in a redox chemical reaction, whereby the oxidizing agent becomes reduced.

As previously stated hereinabove, in the context of the field and art of the present invention, 'whitening teeth' (or teeth whitening) generally refers to application of any number of chemical (e.g., oxidation, reduction) or/and physical (e.g., mechanical, thermal, irradiation, electrical) processes, procedures, techniques, or treatments, performed singly or in combination, which results in returning, or/and providing, white color to teeth (i.e., to a single tooth, or to two or more teeth).

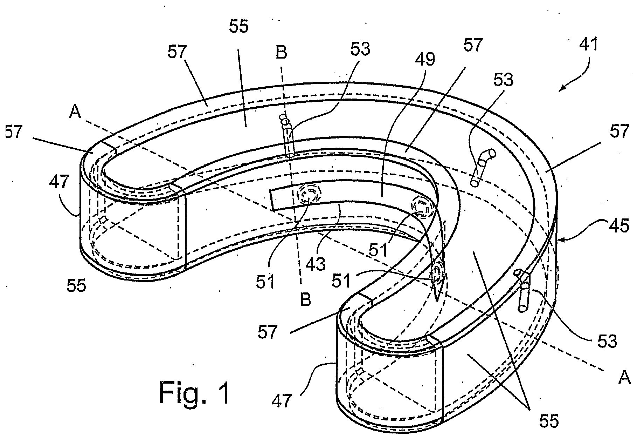

Reference is now made to Fig. 1, which is an isometric view of a device 41, herein, also referred to as electrochemical teeth whitening device 41, which is suitable for electrochemical redox reactions which take place during application of an oxidizing agent to teeth, in accordance with a preferred example useful for understanding the present invention. Device 41 includes a treatment tray is designed to hold an oxidizing agent, for example, either being, or including, a form of hydrogen peroxide, such as a solution form of hydrogen peroxide, for immersion of teeth therein. In a non-limiting manner, some additional examples of an oxidizing agent are selected from the group consisting of a whitening solution, a whitening gel, a solution of or including carbamide peroxide, a gel of or including carbamide peroxide, a solution of or including sodium perborate, and a gel of or including sodium perborate. Device 41 is of a shape similar to typical dental trays, including a back curved wall

43 and a front curved wall 45 joined together at end sections 47. Space or well 55 is configured in between back curved wall 43 and front curved wall 45, and serves for holding or containing a substance, particularly, an oxidizing agent (such as hydrogen peroxide solution or gel, or similar type of oxidizing solution or gel) which is to be applied to the teeth. Back curved wall 43 includes an electrode 49 which is positively charged. Positively charged electrode 49 is comprised of a thin, flexible metal strip attached to an inner portion of back curved wall 43. In a preferred embodiment, positively charged electrode 49 is comprised of stainless steel. In alternative embodiments, electrode 49 is comprised of any inert, electricity conducting, material, such as, platinum, gold, or any other suitable electricity conducting metal, or metal platted with a metal plating. Spacers 51 are positioned along electrode 49, and are designed to preclude contact between electrode 49 and teeth positioned within device 41, while providing contact between the oxidizing agent, such as hydrogen peroxide solution, within device 41 and electrode 49. Spacers 51 are comprised of insulating materials, such as plastics. Front curved wall 45 includes contact electrodes 53, which are negatively charged electrodes, and are designed to be placed in direct contact with at least a part of the front of teeth within device 41. Negatively charged contact electrodes 53 are attached to front curved wall 45 by a spring-like mechanism, ensuring contact between contact electrodes 53 and at least part of the front of the teeth. Preferably, at least three contact electrodes 53 are used, each one designed to contact at least part of the front of a different tooth. The contact area between contact electrodes 53 and the teeth is minimized. In a preferred embodiment, device 41 further includes a sealing lip 57. In alternative embodiments, device 41 is comprised of heatable plastics, which can be formed on the teeth and gums during placement. An adhesive or a repelling substance such as Vaseline(TM) may also be used to further ensure sealing.

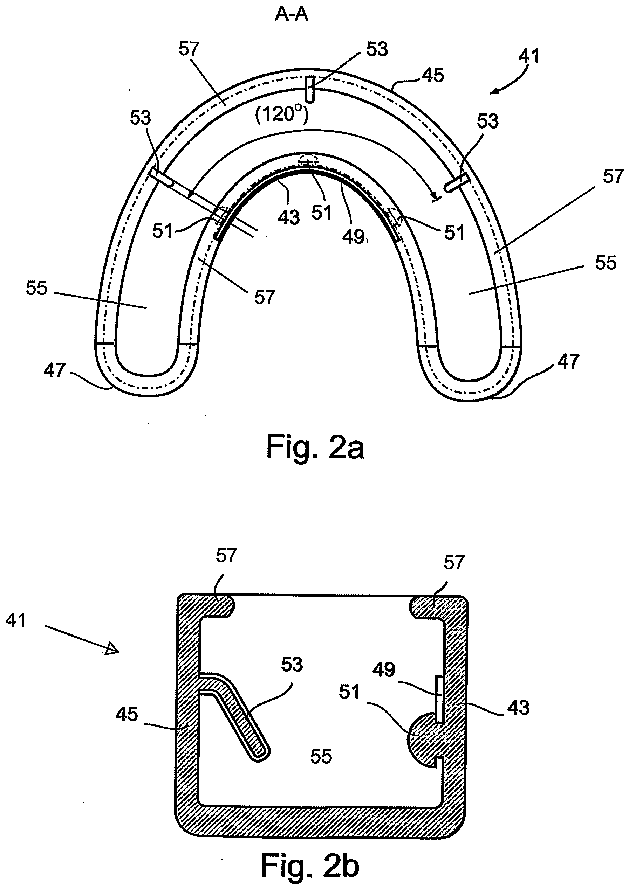

Reference is now made to Figs. 2a and 2b, which are cross sectional illustrations of device 41 along lines A-A and B-B, respectively, illustrated in Fig. 1. As shown in Fig. 2a, spacers 51 may contact the back of the teeth, while (negatively charged) contact electrodes 53 are designed to contact at least part of the front of the teeth. As shown in Fig. 2b, spacers 51 are fixed, and contact electrodes 53 are attached to front curved wall 45 by a spring-like mechanism, for providing spring-like nature and behavior to contact electrodes 53. In the exemplary embodiment shown in Fig. 2b, the spring-like nature and behavior are accomplished by placing contact electrode 53 at an angle, for example, of about 45 degrees. Alternatively, actual springs may used for providing spring-like nature and behavior to contact electrodes 53. In a preferred embodiment, three contact electrodes 53 contact three different teeth. The contact area of contact electrodes 53 is minimal, so as to transmit electrical current to the teeth, while avoiding occurrence of the competitive reaction of water electrolysis (i.e., electrolytic degradation of water).

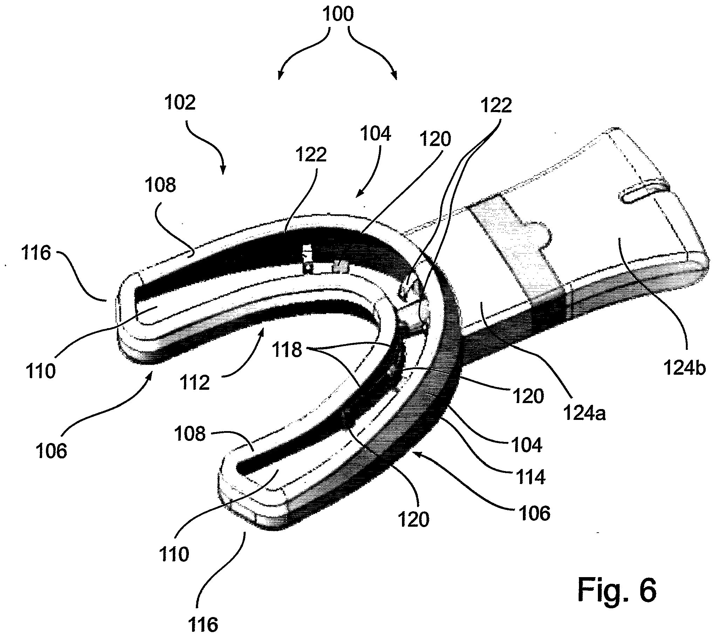

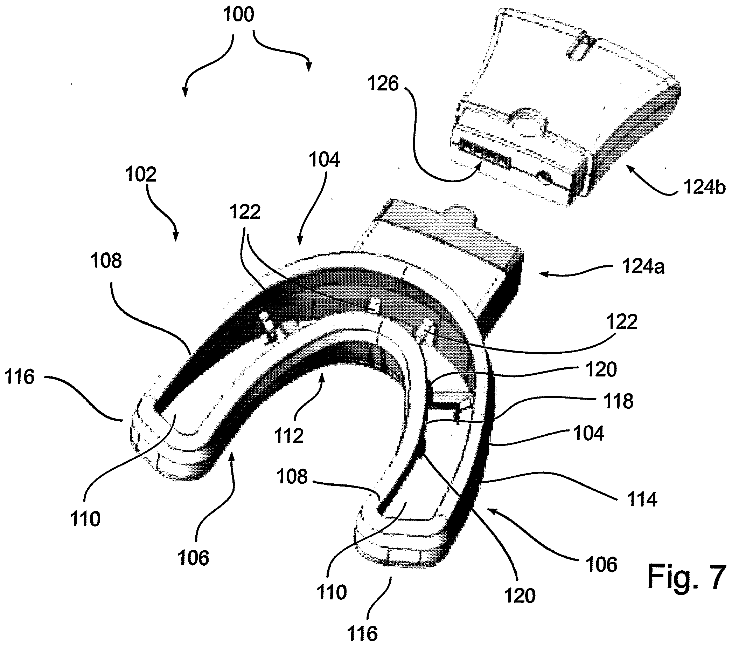

As shown in Figs. 1, 2a, and 2b, electrochemical teeth whitening device 41 includes a sealing lip 57 having an even shape or contour. In another exemplary preferred example of the electrochemical teeth whitening device 41 useful for understanding the present invention, sealing lip 57 features a 'zig-zag' shape or contour, for example, as illustrated in Fig. 8, being a schematic diagram illustrating the electrochemical teeth whitening device 100 shown in Figs. 6 and 7, particularly showing the sealing lip 108 with a 'zig-zag' shape or contour.

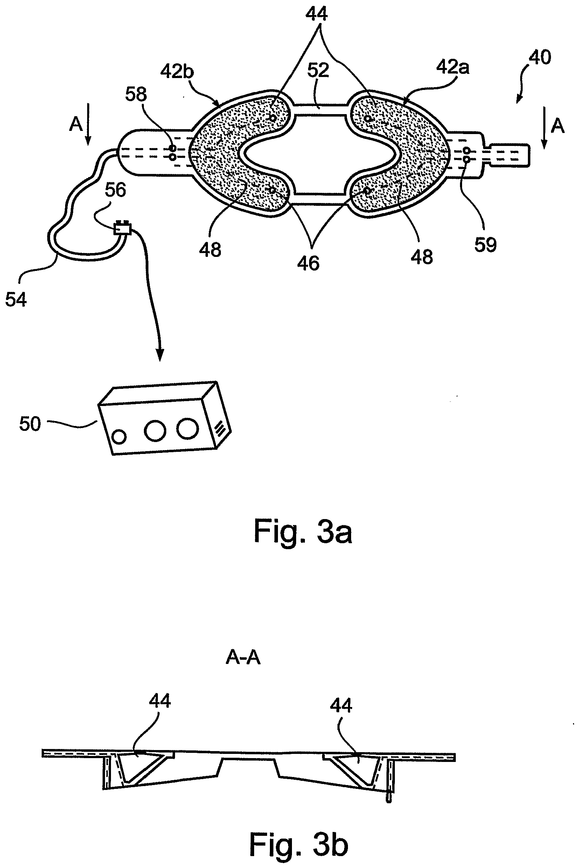

Reference is now made to Figs. 3 a and 3 b, which are an illustration and a cross-sectional view, respectively, of an apparatus 40, for whitening teeth, in accordance with another example useful for understanding the present invention. Apparatus 40 includes a set of trays 42, including an upper tray 42a and a lower tray 42b, each of which has wells 44 for holding an oxidizing agent, for example, either being, or including, a form of hydrogen peroxide, such as a solution form of hydrogen peroxide, for immersion of teeth therein. As shown in Fig. 3a, trays 42 have plugs and electrodes 46 positioned therein, which are connected via conducting wires 48 to a power supply 50. Electrodes are comprised of inert, electricity conducting, material, such as stainless steel, platinum, gold, or any other suitable electricity conducting metal. In a preferred embodiment, trays 42 are comprised of a non-conducting material, such as a biocompatible plastic material. It should be noted that any biocompatible plastic material which does not react with an oxidizing agent, for example, a form of hydrogen peroxide, such as hydrogen peroxide solution, for whitening teeth, may be used as material of construction of trays 42a and 42b.

Trays 42, and wells 44, are sized in accordance with standard sized dental trays. In one embodiment, the upper and the lower trays 42 are connected to each other via a folding bridge 52, which allows both trays 42 to be placed on the teeth simultaneously by folding the trays back in the area of folding bridge 52. An electrical connector 54 connects electrodes 48 to power source 50, via a plug 56, or via any other suitable means. Plug 56 is designed to fit into power source 50. Furthermore, upper and lower trays 42 are connectable to one another via a socket 58 and a socket connector 59. Socket 58 is located on an outer rim of one of trays 42, for example, tray 42b, as shown in Fig. 3a, such that when trays 42 are folded at folding bridge 52, socket connector 59, located on an outer rim of the other tray, for example, tray 42a, as shown in Fig. 3a, connects the two trays 42 and completes the electrochemical circuit. In another embodiment, the trays 42 are not connected to each other.

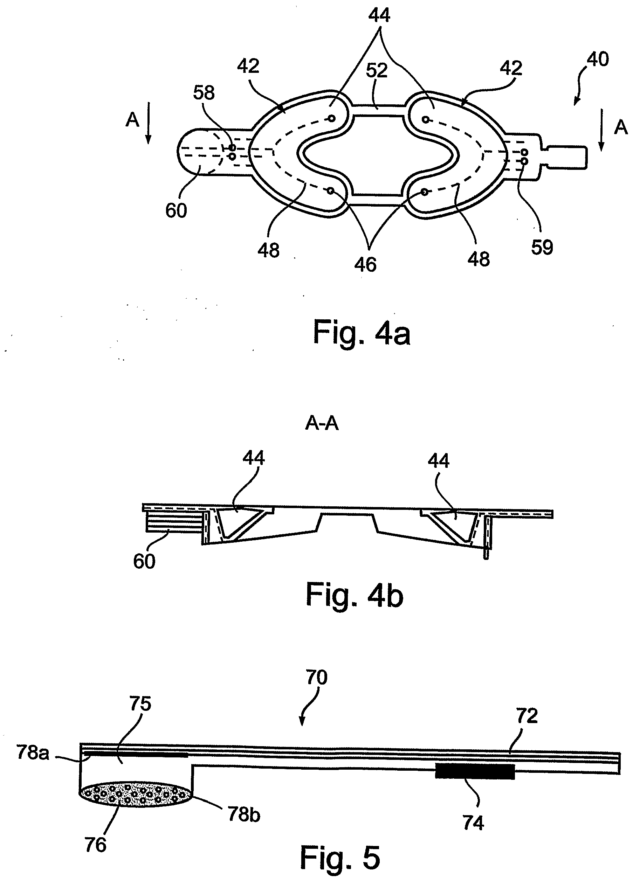

In one embodiment, as shown in Fig. 3a, device 40 is connected to a regular power supply, with a standard AC/DC transformer. In another embodiment, rechargeable batteries (1 - 12 volt) are used. In yet another embodiment, shown in Figs. 4a and 4b, a disposable battery 60 is embedded in device 40, allowing it to be more easily transportable. It should be readily apparent, that in all of the herein illustratively described embodiments/examples useful for understanding the present invention, the electrochemical circuit runs through electrochemical teeth whitening device 41, or 40, and not through any non-intra-oral cavity body part of the user. In this way, oxidizing activity by the oxidizing agent can be increased, thus, enhancing oxidizing agent uptake by the teeth, for whitening teeth. Additionally, in alternative example useful for understanding the present invention, different sized electrochemical teeth whitening devices 41, or 40, are provided for different sized individuals.

Reference is now made to FIG. 5, which is an illustration of an electrochemical teeth whitening toothbrush 70, in accordance with another example useful for understanding the present invention. Toothbrush 70 includes a handle 72 having a battery 74 placed therein, and a head portion 75 at an opposite end thereof. Head portion 75 includes a bristle portion 76 on a lower end thereof, and further has two electrodes 78 - a positively charged electrode 78a on an upper end, and a negatively charged electrode 78b in an area of the bristles. An electrochemical circuit is completed, and electrical current is transmitted, through positively charged electrode 78a, at least part of the tooth which is in contact with bristle portion 76, and negatively charged electrode 78b. Toothbrush 70 can be used with an oxidizing agent, for example, either being, or including, a form of hydrogen peroxide, such as a gel or paste form of hydrogen peroxide, for brushing of teeth therein, for whitening teeth. The electrochemical circuit, and transmitted electrical current, of toothbrush 70 does not run through a non-intra-oral cavity body part of a user.

Figs. 6 and 7 are schematic diagrams illustrating perspective views of another exemplary preferred embodiment of an electrochemical teeth whitening device 100, for electrochemically whitening teeth, showing the replaceable / rechargeable power supply in a connected or closed configuration (Fig. 6), and in a disconnected or open configuration (Fig. 7), in accordance with an example useful for understanding the present invention.

In Figs. 6 and 7, electrochemical teeth whitening device 100 is usable for electrochemical redox reactions involving an oxidizing agent for whitening teeth, in accordance with a preferred example useful for understanding the present invention. Device 100 includes a tray designed to hold an oxidizing agent, for example, either being, or including, a form of hydrogen peroxide, such as a solution form of hydrogen peroxide, for immersion of teeth therein, when the present example is implemented for whitening teeth.

In electrochemical teeth whitening device 100, treatment tray 102 has an upper portion 104 and a lower portion 106. As shown in Figs. 6 and 7, upper portion 104 preferably includes a sealing lip 108, being similar to sealing lip 57 of electrochemical teeth whitening device 41 previously described hereinabove with reference to Figs. 1, 2a, and 2b. Sealing lip 108 is designed to contact the gums, thus, sealing tray 102 onto the gums and substantially preventing leaking out of solution or gel compositions used during the electrochemical whitening of the teeth. As shown in Figs. 6 and 7, sealing lip 57 has an even shape or contour. In another exemplary preferred embodiment of electrochemical teeth whitening device 100, sealing lip 108 features a 'zig-zag' shape or contour, for example, as illustrated in Fig. 8. In electrochemical teeth whitening device 100, lower portion 106 includes a space 110, within which the teeth are appropriately positioned for performing the electrochemical teeth whitening procedure. In one example useful for understanding the present invention, tray 102 is comprised of a plastic material. Alternatively, an adhesive material which is biocompatible (such as, for example, beeswax) may be added to sealing lip 108 to ensure sealing of tray 102 onto the gums.

Electrochemical teeth whitening device 100 is of a shape similar to typical dental trays, including a back curved wall 112 and a front curved wall 114 joined together at end sections 116. Back curved wall 112 includes an electrode 118 which is positively charged. Positively charged electrode 118 is comprised of a thin, flexible metal strip attached to an inner portion of back curved wall 112. In a preferred embodiment, electrode 118 is comprised of stainless steel. In alternative embodiments, electrode 118 is comprised of any inert, electricity conducting, material, such as, platinum, gold, or any other suitable electricity conducting metal, or metal platted with a metal plating. Spacers 120 are positioned along electrode 118, and are designed to preclude contact between electrode 118 and teeth positioned within device 100, while providing contact between the oxidizing agent, such as hydrogen peroxide solution, within device 100 and electrode 118. Spacers 120 are comprised of insulating materials, such as plastics.

Front curved wall 114 includes contact electrodes 122, which are negatively charged, and are designed to be placed in direct contact with the front of teeth within device 100. Negatively charged contact electrodes 122 are attached to front curved wall 114 by a spring-like mechanism, ensuring contact between contact electrodes 122 at least part of and the front of the teeth. Preferably, at least three contact electrodes 122 are used, each one designed to contact at least part of the front of a different tooth. The contact area between contact electrodes 122 and the teeth is minimized. In alternative embodiments, device 100 is comprised of heatable plastics, which can be formed on the teeth and gums during placement. An adhesive or a repelling substance such as Vaseline may also be used to further ensure sealing of treatment tray 102 onto the gums.

In an exemplary preferred embodiment, as shown in Figs. 6 and 7, electrochemical teeth whitening device 100 includes a mobile current supply unit 124b containing a disposable or rechargeable battery operative in a range of between about 0.001 volt and about 12 volts. Mobile current supply unit 124b is operatively (physically and electrically) connectable, via connector assembly 124a, to device 100. Preferably, connector assembly 124a is connected or attached to the central portion of front curved wall 114. Mobile current supply unit 124b is physically and electrically connectable, for example, via a female configured electrical contact assembly 126 (Fig. 7), to a male configured electrical contact or lead assembly (not shown) included as part of connector assembly 124a. Preferably, mobile current supply unit 124b includes a light window or indicator light, for example, as shown by the inserted section located at the outer end of mobile current supply unit 124b, for indicating a condition of 'power on', i.e., when mobile current supply unit 124b actively supplies current to electrochemical teeth whitening device 100, thereby indicating that electrochemical teeth whitening device 100 is in an 'on' mode for electrochemically whitening the teeth.

As stated hereinabove, the method for electrochemically whitening teeth includes the following main procedures, and, components and functionalities thereof: applying a metal salt solution to the teeth, applying an oxidizing agent to the teeth, and transmitting electrical current to the teeth so as to activate and reduce the oxidizing agent for effecting the whitening of the teeth.

Accordingly, the method of electrochemically whitening teeth, in accordance with the electrochemical teeth whitening apparatus described hereinabove is as follows, in accordance with one example useful for understanding the present invention. Initially, as a pre-treatment procedure, teeth are thoroughly cleaned, either by professional cleaning or using a regular toothbrush. Next, teeth are rinsed with distilled water.

Then, a metal catalyst solution, which functions as an activating solution, is prepared by dissolving different amounts of metal, or metal salt, solutions, such as of silver, copper, titanium, or palladium, in an amount of distilled water such that the concentration of the metal is in a range of between about 0.01 % and about 3 % by weight. In alternative exemplary preferred embodiments, the metal, or metal salt, solution can include silver nitrate, palladium hydroxide, palladium chloride, or copper chloride, salts, or any other suitable activator. In one specific example, 100 mg of silver nitrate is dissolved in 10 ml distilled water. In yet another specific example, 50 mg of copper chloride is dissolved in 10 ml of distilled water. A pre-treatment tray, such as pre-treatment tray 30 shown in Fig. 3 and described in

PCT International Publication No. WO 2005/062710, entitled: "Ion Exchange Dental Device And Method", assigned to the same applicant of the present invention, is placed on the teeth, preferably, in such a way that all of the teeth are in contact with an absorbent material, for example, a sponge. A sealing lip of the pre-treatment tray is placed in contact with the gums to seal the pre-treatment tray and prevent leaking of solution into the mouth. A pre-measured amount of the metal catalyst (activating) solution is then introduced into the pre-treatment tray by injection or any other filling procedure, such that full contact between the teeth and the solution is obtained, for a time period of approximately 1 minute. Alternatively, a known amount of metal catalyst (activating) solution is placed into the pre-treatment tray prior to placing the tray in the mouth. After pre-treatment, the pre-treatment tray is removed from the mouth, taking care to avoid spilling of the activating solution into the mouth. The teeth are then rinsed with distilled water again.

After pre-treatment of the teeth, whitening treatment is commenced. An oxidizing agent, for example, a tooth whitening agent, such as a commercial tooth whitening gel or solution, including hydrogen peroxide, or any other suitable oxidizing agent (e.g., ion, free radical donor) is placed in the tray of any one of the above illustratively described embodiments of the electrochemical teeth whitening device, and then the tray is placed on the teeth in such a manner that all of the teeth are in contact with the oxidizing agent (whitening agent).In a non-limiting manner, additional examples of an oxidizing agent are selected from the group consisting of a whitening solution, a whitening gel, a solution of or including carbamide peroxide, a gel of or including carbamide peroxide, a solution of or including sodium perborate, and a gel of or including sodium perborate. Any number of these exemplary oxidizing agents may be used for implementing the present invention.

The electrochemical circuit of the electrochemical dental treatment device is then activated, via a power supply, using a voltage in a range of between about 0.001 volt and about 12 volts. In a preferred embodiment, the voltage is in a range of between about 3 volts and about 9 volts. The electrochemical circuit remains open for a predetermined period of time, for example, in a range of between about 3 minutes and about 20 minutes. Preferably, the electrical current passing through the electrochemical circuit does not exceed about 30 milliamps (mA). Preferably, transmitting electrical current to the teeth so as to activate and reduce the oxidizing agent for effecting the whitening of the teeth, is done for the electrical current in a range of between about 0.001 milliamp (mA) and about 30 milliamps (mA), and more preferably, in a range of between about 5 milliamp (mA) and about 15 milliamps (mA). Preferably, transmitting the electrical current is done by using a rechargeable battery, or alternatively, by using a disposable battery embedded into the electrochemical teeth whitening device which is used for applying the oxidizing agent.

Additional objects, advantages, and novel features useful for understanding the present invention will become apparent to one ordinarily skilled in the art upon examination of the following example, which is not intended to be limiting. Additionally, each of the various examples useful for understanding the present invention as delineated hereinabove finds experimental support in the following example.

### EXAMPLE

Reference is now made to the following example, which together with the above description, illustrate an example useful for understanding the invention in a non-limiting fashion.

### EXAMPLE

Electrochemically Whitening Teeth

This is an example of implementing the hereinabove illustratively described example useful for understanding the invention of electrochemically whitening teeth.

### EXPERIMENTAL PROCEDURE

Fresh extracted human teeth were washed with tooth paste for decontamination prior to use. The teeth were divided into three groups, i.e., Groups A, B, and C. Each group of teeth was immersed in 19 % (weight/weight) hydrogen peroxide gel for 15 minutes as follows:
1. cleaning with tooth paste.
2. rinse with deionized water.
3. tooth color determination.
4. with or without pretreatment (see groups).
5. deionized water.
6. 15 minutes treatment with 19 % (weight/weight) hydrogen peroxide gel.
7. rinse with deionized water.

## Claims

1. A device (100) for whitening teeth, the device (100) comprising:
a dental tray (102) suitable for applying an oxidizing agent to the teeth, said dental tray (102) having a first end (112) and a second end (114);
a first electrode (118) attached to said first end (112) of said dental tray (102);
a second electrode (122) attached to said second end (114) of said dental tray, wherein said first electrode (118) and said second electrode (122) are configured to transmit electrical current through said dental tray (102); and
the oxidizing agent suitable for placement within said dental tray (102),
wherein said oxidizing agent is configured to undergo activation and reduction upon transmission of said electrical current through said oxidizing agent, thereby effecting the whitening of the teeth;
wherein said dental tray (102) includes an upper portion (104) comprising a sealing lip (108) having a zig-zag shape or contour.

2. The device (40; 41; 100) of claim 1, wherein said oxidizing agent is a teeth whitening agent.

3. The device (100) of claim 2, wherein said teeth whitening agent includes hydrogen peroxide.

4. The device (100) of claim 1, wherein said oxidizing agent is selected from the group consisting of a whitening solution, a whitening gel, a solution of or including carbamide peroxide, a gel of or including carbamide peroxide, a solution of or including sodium perborate, and a gel of or including sodium perborate.

5. The device (100) of claim 1, wherein said first end (112) is a back curved wall and said first electrode (118) is positively charged.

6. The device (100) of claim 1, wherein said first electrode (118) is a flat metal strip.

7. The device (100) of claim 1, wherein said second end (114) is a front wall and said second electrode (122) is negatively charged.

8. The device (100) of claim 1, wherein said second electrode (122) further comprises a spring mechanism.

9. The device (100) of claim 1, further comprising a power supply.

10. The device (100) of claim 9, wherein said power supply is an external power source (50).

11. The device (100) of claim 9, wherein said power supply is a rechargeable battery (124b).

12. The device (100) of claim 9, wherein said power supply is a disposable battery (60) embedded within said dental tray (102).

## Patentansprüche

1. Einrichtung (100) zum Bleichen von Zähnen, wobei die Einrichtung (100) umfasst:
eine Zahnschiene (102), die dazu geeignet ist, ein Oxidationsmittel auf die Zähne aufzutragen, wobei die Zahnschiene (102) ein erstes Ende (112) und ein zweites Ende (114) aufweist;
eine erste Elektrode (118), die am ersten Ende (112) der Zahnschiene (102) angebracht ist;
eine zweite Elektrode (122), die am zweiten Ende (114) der Zahnschiene angebracht ist, wobei die erste Elektrode (118) und die zweite Elektrode (122) so konfiguriert sind, dass sie elektrischen Strom durch die Zahnschiene (102) übertragen; und
wobei das Oxidationsmittel dazu geeignet ist, innerhalb der Zahnschiene (102) platziert zu werden, wobei das Oxidationsmittel so konfiguriert ist, dass es bei Übertragung des elektrischen Stroms durch das Oxidationsmittel aktiviert und verringert wird, wodurch die Bleichung der Zähne bewirkt wird;
wobei die Zahnschiene (102) einen oberen Abschnitt (104) umfasst, der eine Dichtungslippe (108) mit Zickzackform oder -kontur umfasst.

2. Einrichtung (40; 41; 100) nach Anspruch 1, wobei das Oxidationsmittel ein Zahnbleichungsmittel ist.

3. Einrichtung (100) nach Anspruch 2, wobei das Zahnbleichungsmittel Wasserstoffperoxid umfasst.

4. Einrichtung (100) nach Anspruch 1, wobei das Oxidationsmittel aus der Gruppe ausgewählt ist, bestehend aus einer Bleichungslösung, einem Bleichungsgel, einer Carbamidperoxidlösung oder Lösung, die Carbamidperoxid umfasst, einem Carbamidperoxidgel oder Gel, das Carbamidperoxid umfasst, einer Natriumperboratlösung oder Lösung, die Natriumperborat umfasst, und einem Natriumperboratgel oder Gel, das Natriumperborat umfasst.

5. Einrichtung (100) nach Anspruch 1, wobei das erste Ende (112) eine gekrümmte hintere Wand ist und die erste Elektrode (118) positiv geladen ist.

6. Einrichtung (100) nach Anspruch 1, wobei die erste Elektrode (118) ein flacher Metallstreifen ist.

7. Einrichtung (100) nach Anspruch 1, wobei das zweite Ende (114) eine vordere Wand ist und die zweite Elektrode (122) negativ geladen ist.

8. Einrichtung (100) nach Anspruch 1, wobei die zweite Elektrode (122) ferner einen Federmechanismus umfasst.

9. Einrichtung (100) nach Anspruch 1, die ferner eine Energieversorgung umfasst.

10. Einrichtung (100) nach Anspruch 9, wobei die Energieversorgung eine externe Energiequelle (50) ist.

11. Einrichtung (100) nach Anspruch 9, wobei die Energieversorgung eine wiederaufladbare Batterie (124b) ist.

12. Einrichtung (100) nach Anspruch 9, wobei die Energieversorgung eine Einwegbatterie (60) ist, die in der Zahnschiene (102) eingebettet ist.

## Revendications

1. Dispositif (100) pour le blanchiment des dents, le dispositif (100) comprenant :
un plateau dentaire (102) approprié pour appliquer un agent oxydant sur les dents, ledit plateau dentaire (102) ayant une première extrémité (112) et une seconde extrémité (114) ;
une première électrode (118) fixée à ladite première extrémité (112) dudit plateau dentaire (102) ;
une seconde électrode (122) fixée à ladite seconde extrémité (114) dudit plateau dentaire, dans lequel ladite première électrode (118) et ladite seconde électrode (122) sont conçues pour transmettre un courant électrique à travers ledit plateau dentaire (102) ; et
l'agent oxydant approprié pour un placement au sein dudit plateau dentaire (102), dans lequel ledit agent oxydant est conçu pour subir une activation et une réduction lors de la transmission dudit courant électrique par l'intermédiaire dudit agent oxydant, effectuant ainsi le blanchiment des dents ;
dans lequel ledit plateau dentaire (102) comprend une partie supérieure (104) comprenant une lèvre d'étanchéité (108) ayant une forme ou un contour en zig-zag.

2. Dispositif (40 ; 41 ; 100) selon la revendication 1, dans lequel ledit agent oxydant est un agent pour le blanchiment des dents.

3. Dispositif (100) selon la revendication 2, dans lequel ledit agent de blanchiment des dents comprend du peroxyde d'hydrogène.

4. Dispositif (100) selon la revendication 1, dans lequel ledit agent oxydant est choisi dans le groupe constitué par une solution de blanchiment, un gel de blanchiment, une solution de ou comprenant du peroxyde de carbamide, un gel de ou comprenant du peroxyde de carbamide, une solution de ou comprenant du perborate sodium, et un gel de ou comprenant du perborate de sodium.

5. Dispositif (100) selon la revendication 1, dans lequel ladite première extrémité (112) est une paroi arrière courbée et ladite première électrode (118) est chargée positivement.

6. Dispositif (100) selon la revendication 1, dans lequel ladite première électrode (118) est une bande de métal plate.

7. Dispositif (100) selon la revendication 1, dans lequel ladite seconde extrémité (114) est une paroi avant et ladite seconde électrode (122) est chargée négativement.

8. Dispositif (100) selon la revendication 1, dans lequel ladite seconde électrode (122) comprend en outre un mécanisme à ressort.

9. Dispositif (100) selon la revendication 1, comprenant en outre une alimentation.

10. Dispositif (100) selon la revendication 9, dans lequel ladite alimentation est une source d'alimentation externe (50).

11. Dispositif (100) selon la revendication 9, dans lequel ladite alimentation est une batterie rechargeable (124b).

12. Dispositif (100) selon la revendication 9, dans lequel ladite alimentation est une batterie jetable (60) intégrée dans ledit plateau dentaire (102).
